# FASCICULE DE BREVET EUROPEEN

(11) **EP 0 810 986 B1**
(45) Date de publication et mention de la délivrance du brevet: **15.09.1999**
(21) Numéro de dépôt: 96939252.1
(22) Date de dépôt: 06.12.1996
(51) Int. Cl.: C07C 45/34, C07C 29/50, C07C 49/653, C07C 35/36

(54) **PROCEDE POUR LA PREPARATION DE LA NOOTKATONE**
VERFAHREN ZUR HERSTELLUNG VON NOOTKATON
METHOD FOR PREPARING NOOTKATONE

(30) Priorité: 18.12.1995 CH 356295
(43) Date de publication de la demande: 10.12.1997
(73) Titulaire: FIRMENICH SA, 1211 Genève 8 (CH)
(72) Inventeur: MULLER, Bernard, CH-1292 Chambésy (CH); DEAN, Christopher, CH-1212 Grand-Lancy (CH); SCHMIDT, Christian, D-22587 Hamburg (DE); KUHN, Jean-Charles, CH-1203 Genève (CH)
(74) Mandataire: Salvaterra-Garcia, Maria de Lurdes
(86) Numéro de dépôt international: IB9601371
(87) Numéro de publication internationale: WO9722575

(56) Documents cités:
- EP-A- 0 198 351
- US-A- 3 887 622
- PATENT ABSTRACTS OF JAPAN vol. 008, no. 118 (C-226), 31 Mai 1984 & JP 59 031728 A (HASEGAWA KORYO KK), 20 Février 1984,
- CHEMIKER-ZEITUNG, vol. 115, no. 12, Décembre 1991, pages 356-360, XP000650823 WILLERSHAUSEN, H. & GRAF, H.: "Metallkatalytische Transformation von Valenzen zu Nootkaton" & "Enzymatische Transformation von Valenzen zu Nootkaton"

## Description

La présente invention a trait au domaine de la synthèse organique et plus particulièrement à un procédé pour la préparation de la nootkatone ou du nootkatol, ou des mélanges de ces composés, par oxydation du valencène, caractérisé en ce qu'on expose ce dernier à une atmosphère d'oxygène dans un milieu de réaction approprié et en présence d'un hydropéroxyde d'un acide gras insaturé et en ce que, le cas échéant, on sépare la nootkatone et/ou le nootkatol du mélange réactionnel.

La nootkatone, ou 4,4a,5,6,7,8-hexahydro-6-isopropényl-4,4a-diméthyl-2(3H)-naphtalénone, le nootkatol, ou 2,3,4,4a,5,6,7,8-octahydro-6-isopropényl-4,4a-diméthyl-2-naphtalénol, et le valencène, ou 1,2,3,5,6,7,8,8a-octahydro-7-isopropényl-1,8a-diméthylnaphtalène, utilisé comme produit de départ dans le procédé de l'invention, sont des composés d'origine naturelle, à savoir des constituants naturels d'huiles d'agrumes, de pamplemousse parmi d'autres, dans lesquels ils sont présents sous une forme optiquement active.

En raison de ses excellentes qualités organoleptiques et notamment de son goût typique de pamplemousse, la nootkatone est un ingrédient d'emploi courant dans la parfumerie et dans l'industrie des arômes. D'autre part, en conséquence des normes législatives actuellement en vigueur dans la plupart des pays, relatives à l'utilisation d'additifs alimentaires, il est d'une importance capitale de pouvoir disposer d'un procédé de synthèse de ce composé qui soit, non seulement rentable et applicable à l'échelle industrielle, mais, en plus, susceptible de fournir ce produit avec une qualité pouvant satisfaire les critères établis quant au caractère naturel de son origine. Or, actuellement, une telle qualité ne peut être obtenue que par extraction des produits naturels contenant la nootkatone, notamment le pamplemousse, une méthode qui n'est guère économique.

### Technique antérieure

Nombreuses sont les méthodes décrites jusqu'à maintenant pour l'obtention de la nootkatone par oxydation du valencène. On peut citer, parmi les plus anciennes, l'oxydation du valencène à l'aide du chromate de tert-butyle (voir, par exemple, G.L.K. Hunter et al., dans J. Food Sci. 1965, 30, 876) ou de peracétate de tert-butyle (voir C.W. Wilson et al., J. Agric. Food Chem. 1978, 26, 1430).

On connaît également d'autres procédés chimiques à plusieurs étapes, à partir du nootkatène (voir, par exemple, le brevet canadien n°901601), du cyclohexène (voir M. Pesaro et al., Chem. Comm. 1968, 19, 1152) ou encore du cyclohexadiène (voir A.J. Birch, J. Agr. Food Chem. 1974, 22, 162).

La bioconversion du valencène en nootkatone, catalysée par des microorganismes (voir R.S. Dhavlikar et al., Dragoco Rep. 1973, 12, 251) ou à partir de cultures de cellules végétales (F.Drawert et al., Plant Cell Reports 1984, 3, 37) a également été étudiée, mais ces deux méthodes fournissent des rendements en nootkatone beaucoup trop pauvres pour que leur exploitation commerciale soit envisageable.

Le brevet américain US 3,887,622, quant à lui, divulgue un procédé de préparation de cétones de sesquiterpènes à partir des sesquiterpènes correspondants ayant une liaison oléfinique en position endocyclique, par l'oxydation de ces sesquiterpènes avec du péroxyde d'hydrogène en présence du formate d'un alkyle inférieur. Dans les exemples de ce brevet, la préparation de l'isolongifolanone, de la cédranone et de la thuyopsane est décrite.

La demande européenne EP-A-198 351 décrit également la préparation de plusieurs cétones de sesquiterpènes, dont la nootkatone, par l'oxydation du terpène correspondant. Cette fois-ci, l'oxydation est exécutée en présence d'une N-hydroxydicarboximide dans une cétone ou un ester inerte à l'aide de l'oxygène ou d'un gaz contenant l'oxygène.

De la référence Patent Abstracts of Japan, vol. 008, n° 118 (C-226), on connaît un autre procédé de préparation de la nootkatone, ce procédé faisant recours à l'oxydation du valencène avec de l'oxygène ou un gaz contenant ce dernier, en présence d'un composé organométallique d'un métal du groupe VIII du tableau périodique de Mendeleev.

Il convient encore de citer deux publications de H. Willershausen et H. Graf dans Chemiker-Zeitung, vol. 115, n°12, pages 356 à 358 et 358 à 360, divulguant deux procédés de préparation de la nootkatone par l'oxydation du valencène. L'un de ces procédés comprend une oxydation par l'oxygène en présence de sels de Fe, Ni, Zn, Co, Mn et Cu, l'autre de ces procédés est un procédé enzymatique qui fait recours aux enzymes ligninperoxidase et lactoperoxidase.

Ainsi, tous ces procédés connus se révèlent soit inadaptés à l'obtention d'un produit satisfaisant les critères établis quant à son caractère naturel, soit trop compliqués et trop onéreux, donc inadaptés à une exploitation à l'échelle industrielle.

### Description de l'invention

La présente invention apporte justement une solution avantageuse au problème de la synthèse de la nootkatone.

Le procédé de l'invention cité plus haut est un procédé à une seule étape, d'une exécution extrêmement simple, qui peut fournir la nootkatone avec des rendements très avantageux et une qualité conforme aux normes requises pour une utilisation dans des produits alimentaires.

Nous avons maintenant découvert qu'il était désormais possible d'obtenir ce produit par simple oxydation du valencène en présence d'un hydropéroxyde d'un acide gras insaturé. Cette transformation se déroule sous atmosphère d'oxygène et dans un milieu réactionnel approprié, c'est-à-dire propice à la formation du produit désiré avec des rendements utiles pour une exploitation rentable et à large échelle.

Par ailleurs, comme il est cité plus haut, le procédé de l'invention permet aussi d'obtenir le nootkatol. Il s'agit également d'un composé connu, cité notamment à plusieurs reprises en tant que précurseur de la nootkatone, aussi bien dans des procédés de synthèse de celle-ci (voir, par exemple, GB 1 299 299), que lors de sa formation à partir de cultures de cellules végétales (F. Drawert et al., réf. citée). Il possède des propriétés organoleptiques distinctes de celles de la nootkatone, mais également avec des tonalités de type agrumes.

Selon l'invention, on obtiendra typiquement en tant que produit de l'oxydation un mélange de nootkatone et de nootkatol, où les proportions relatives de ces deux produits peuvent être variées en fonction des conditions de réaction. Etant donné que ces deux composants possèdent des propriétés organoleptiques de base similaires, les mélanges ainsi obtenus peuvent être utilisés en tant que tels pour des applications dans le domaine des arômes ou de la parfumerie. D'autre part, si désiré, on peut séparer ces composés du produit de la réaction à l'aide des techniques usuelles, telles que chromatographie ou distillation.

L'oxydation qui caractérise le procédé de l'invention a lieu sous atmosphère d'oxygène. Celle-ci peut être constituée par de l'air, mais il a été constaté que de meilleurs rendements en produit final pouvaient être obtenus lorsqu'on employait une atmosphère enrichie en oxygène, par exemple un mélange d'air et oxygène. Selon un mode d'exécution plus préférentiel, on utilisera une atmosphère d'oxygène pur.

Le milieu de réaction dans le procédé de l'invention sera typiquement un milieu aqueux, contenant le substrat et l'hydropéroxyde d'acide gras insaturé, des moyens étant prévus pour permettre le contrôle de la température et du pH du milieu pendant la durée de la réaction.

Selon l'invention, l'hydropéroxyde d'acide gras insaturé peut être ajouté directement au milieu de réaction, sa préparation pouvant être effectuée au préalable par autooxydation ou photooxydation, ou oxydation catalysée enzymatiquement ou chimiquement, de l'acide gras correspondant. Alternativement, selon un mode d'exécution préféré de l'invention, l'hydropéroxyde susmentionné peut être engendré in situ dans le milieu de réaction, à partir de l'acide gras insaturé approprié. Ce dernier sera alors sélectionné parmi les acides gras susceptibles de former un ou plusieurs hydropéroxydes dans les conditions de la réaction. A cet effet, on peut citer, par exemple, les acides linoléique ou linolénique, ou encore les précurseurs d'origine naturelle de ces acides tels que les hydrolysats d'huile de lin ou de tournesol. Les hydrolysats d'huiles de poissons, ou les acides gras polyinsaturés y contenus, peuvent également être utilisés. D'excellents rendements en produit final désiré ont été obtenus, par exemple, avec l'acide linoléique ou ses hydropéroxydes, notamment l'acide 13-hydropéroxylinoléique.

La formation de l'hydropéroxyde peut également avoir lieu à partir d'un acide gras monoinsaturé, par exemple l'acide oléique.

Lorsque l'hydropéroxyde est engendré in situ à partir d'un acide gras polyinsaturé, et selon un mode d'exécution préférentiel du procédé de l'invention, on ajoute une lipoxygénase au milieu de réaction. Nous avons observé que l'addition d'une lipoxygénase augmentait l'efficacité du procédé en favorisant une oxydation plus rapide de l'acide gras susmentionné et, ainsi une formation accélérée de l'hydropéroxyde qui oxyde le valencène. La lypoxygénase peut être ajoutée, par exemple, sous forme de farine de soja, obtenue typiquement par broyage de grains de soja. Lorsque la farine de soja n'a pas été dégraissée au préalable, elle présente l'avantage supplémentaire d'apporter des acides gras polyinsaturés capables de former des hydropéroxydes dans les conditions de la réaction.

Il a cependant été constaté que l'utilisation de la lipoxygénase sous une forme purifiée permettait d'obtenir des rendements en produit final encore plus avantageux et évitait les problèmes éventuels liés à une viscosité trop importante du milieu de réaction. On peut ainsi employer une lipoxygénase de la farine de soja, parmi celles qui sont disponibles dans le commerce sous une forme purifiée et, typiquement, lyophilisée. Les exemples décrits plus loin citent des lipoxygénases convenant parfaitement aux buts de l'invention. Des lipoxygénases d'autres origines que la farine de soja peuvent également être utilisées.

Par ailleurs, il a été observé que l'oxydation du valencène pouvait également être accélérée par addition au milieu de réaction contenant l'acide gras insaturé d'un catalyseur susceptible de promouvoir la formation de radicaux, par exemple du cuivre en poudre, du stéarate de cuivre ou encore du naphténate de cobalt.

La température à laquelle a lieu l'oxydation du valencène peut varier dans une gamme de valeurs assez large. On peut citer à cet effet des températures variant entre 20° et 80°C. Il a été constaté, avec surprise, que des températures de l'ordre de 50° ou plus donnaient des résultats particulièrement avantageux.

La réaction peut être effectuée à une valeur du pH comprise entre environ 5,0 et 10. Nous avons observé que le pH du milieu pouvait avoir une influence sur la composition du produit de la réaction et favoriser la formation de l'un ou l'autre des produits désirés, à savoir la nootkatone et le nootkatol. D'autre part, lorsque l'on ajoute une lipoxygénase, le choix du pH peut également être fait en fonction de la source de ladite lipoxygénase.

Comme il ressort des exemples présentés plus loin, pour les lipoxygénases de farine de soja, les rendements les meilleurs en nootkatone ont été obtenus à des valeurs du pH compris entre 7,5 et 9,5, alors que des valeurs de pH inférieures permettaient d'obtenir une proportion plus importante de nootkatol. Il est ainsi possible d'obtenir essentiellement de la nootkatone en effectuant la réaction à des valeurs de pH de l'ordre de 7,5 à 9,5 et plus préférentiellement à un pH d'environ 8, alors qu'un pH plus acide, permet d'obtenir une quantité accrue de nootkatol dans le mélange de la réaction. Ceci présente l'avantage de permettre d'éviter l'étape de séparation selon le procédé de l'invention, tout en obtenant l'un ou l'autre de ces composés sous une forme presque pure, ou en tout cas en quantité fortement prépondérante, notamment en ce qui concerne la nootkatone. Comme il a été cité précédemment, les mélanges de nootkatol et de nootkatone directement obtenus de la réaction sont des ingrédients aromatisants utiles en tant que tels, leurs qualités organoleptiques n'étant pas fortement dépendantes des proportions relatives de ces deux composants. Il est clair cependant que leurs propriétés varieront quelque peu en fonction de ces proportions relatives. Grâce au procédé de l'invention, on peut ainsi obtenir un large éventail de produits, incluant les deux composés susmentionnés à l'état essentiellement pur, ainsi que tous leurs mélanges contenant les proportions relatives les plus variées de ces deux composants.

Il convient aussi de noter que l'on peut obtenir des produits de réaction contenant une certaine quantité de produit de départ, à savoir le valencène, ceci dépendant bien entendu du temps de réaction. Le valencène peut être séparé du mélange réactionnel comme il est décrit en détail dans les exemples. Il ne nuit cependant nullement aux qualités organoleptiques de ce mélange, de sorte que cette séparation peut également être superflue.

Un autre sous-produit pouvant se former dans les conditions de la réaction, mais en quantités moindres, typiquement inférieures à 10% en poids, par rapport au poids du produit réactionnel, est l'époxyde du valencène, ou 1,8a-époxy-6-isopropényl-4,4a-diméthylnaphtalène.

Quoique l'on ait constaté que de meilleurs rendements en nootkatone par exemple étaient réalisés lorsque l'on maintenait le pH constant et compris entre 8 et 9 pendant toute la durée de la réaction, des résultats satisfaisants ont également été obtenus lorsque le pH du milieu était fixé au départ de la réaction à une valeur de l'ordre indiquée ci-dessus, mais n'était pas ajusté ensuite pendant la durée de la réaction.

Selon un mode d'exécution plus particulièrement préféré du procédé de l'invention, l'oxydation du valencène est effectuée sous atmosphère d'oxygène pur et à une température comprise entre 50 et 80°C, le pH du milieu de réaction étant maintenu à une valeur comprise entre 8 et 9,5 pendant toute la durée de la réaction.

L'invention fournit ainsi un procédé permettant d'obtenir des ingrédients aromatisants utiles de qualité variée, composés de nootkatone ou de nootkatol à l'état essentiellement pur, de leurs mélanges, ou encore de mélanges de ces composants avec le valencène. Par la mise en oeuvre de ce procédé, on a pu obtenir la nootkatone dans des concentrations de l'ordre d'au moins 20 à 30 g par kg de mélange réactionnel, et pouvant aller dans les meilleurs cas jusqu'à 60 g/kg ou même plus.

L'invention sera maintenant décrite plus en détail à l'aide des exemples suivants, dans lesquels les températures sont indiquées en degrés Celsius et les abréviations ont le sens usuel dans l'art.

### Manières de réaliser l'invention

### Exemple 1

On a effectué l'oxydation du valencène en suivant la méthode générale décrite ci-après.

### Méthode générale

Un mélange de tampon de phosphate sodique à pH 8,5, de valencène et d'acide gras insaturé dans la proportion désirée est doucement agité dans un ballon à 6 cols équipé d'un agitateur, d'un thermomètre, d'un dispositif de contrôle du pH (PHstat), d'une ampoule d'introduction et d'une entrée d'air et/ou oxygène. Lorsqu'on a utilisé de l'oxygène pur, comme dans le cas de la plupart des essais cités au Tableau présenté plus loin, on a dégazé d'abord le mélange et purgé trois ou quatre fois à vide, ensuite remplacé avec une atmosphère d'oxygène et augmenté la vitesse de l'agitateur à 750 rpm. Le cas échéant, on ajoute d'une seule fois la lipoxygénase contenue dans l'ampoule d'introduction, éventuellement en solution dans le tampon.
On maintient une légère surpression d'oxygène pendant toute la durée de la réaction, de façon à permettre la collection d'aliquotes pour analyse et suite de la réaction, sans contamination d'air. Si désiré, on maintient le pH de la réaction à une valeur constante par addition automatique de solution aqueuse de NaOH. Après le temps de réaction désiré, si nécessaire le mélange est refroidi à température ambiante, ensuite extrait à l'éther (3 x 200 ml), lavé avec une solution aqueuse de NaOH à 10% et ensuite à neutralité avec saumure. Les phases organiques combinées sont séchées sur Na₂SO₄ et concentrées sous vide. Le mélange brut ainsi obtenu est ensuite analysé par chromatographie. Il contient typiquement du nootkatol, de la nootkatone et du valencène non réagi, et éventuellement des quantités moindres de l'époxyde de ce dernier.

Le valencène peut être facilement séparé du produit de réaction par traitement avec silica gel, comme suit: on dissout le produit dans l'hexane et on ajoute à la solution du Kieselgel 60, en agitant pendant environ 5 minutes. La suspension ainsi obtenue est filtrée sur un filtre en verre fritté. Le silica gel est ensuite lavé à l'hexane (2 fois). Les extraits combinés d'hexane, une fois concentrés sous vide, fournissent le valencène désiré.

La nootkatone et le nootkatol sont séparés du mélange restant par chromatographie sur silica gel, ce mélange étant obtenu par désorption du silica gel à l'éther.

Les matières de départ utilisées selon la méthode décrite ci-dessus avaient les caractéristiques suivantes :
- (+)-Valencène :: pureté CG 79,5 - 85% ; [α]²⁰_{D} = +96,5 - +108,8° ; préparé par distillation fractionnée de valencène obtenu par extraction d'agrumes (origine :
Firmenich Citrus Center, Floride, USA)
- Lipoxygénase :: Lipoxydase Type I-S ; origine Sigma Chem. # L 8383
Lipoxydase Type I-B ; origine Sigma Chem. # L 7395
- Farine de soja:: fraîchement obtenue par broyage de grains de soja à l'aide d'un broyeur de type Urschel Comitrol équipé d'une tête de type "di-O-cut".
- Farine de soja dégraissée :: Type I ; pas torréfiée ; origine Sigma Chem. # S 9633
- Acides gras :: Nouracid HE-30 ; origine AKZO Nobel, contenant 64,5% d'acide linoléique (hydrolysat d'huile de tournesol)
Acide linoléique pur (origine : Fluka) ou technique (Nouracid LE-80 ;
origine: AKZO Nobel ; contenant 48,2% acide linolénique + 16,3% acide linoléique)
Acide oléique (origine : Fluka; 65%)

### Méthodes analytiques

Les aliquotes collectés sont traités comme suit: à 500 mg de mélange réactionnel à analyser on ajoute 0,4g de NaCl et on extrait ensuite avec 1 ml d'éther. Après centrifugation, la couche organique est injectée dans l'appareil de chromatographie gazeuse, sous les conditions suivantes :
Colonne Spwax 10 ; 0,53 d.i., 15 m longueur, 100° pendant 1 min, et après jusqu'à 200°, à 5°/min.
Colonne Spb-1 ; 0,53 d.i., 15 m longueur, 100° pendant 1 min, puis jusqu'à 160°, à 5°/min.

Selon un essai typique, on a procédé comme il est décrit ci-dessus, en utilisant 50 g de tampon de phosphate sodique à pH 8,5 (0,1 M), 10 g de valencène, 4,0 g d'acide gras et 600 mg de lipoxygénase Type I-S dans 5 g de tampon de phosphate sodique à pH 8,5. La réaction a été conduite en atmosphère d'oxygène pur, en maintenant la température à 60°. Après 48 h de réaction et le traitement décrit plus haut, on a obtenu 9,41 g d'un produit dont l'analyse chromatographique a montré qu'il contenait 20,2% de nootkatone, 10,1% de nootkatol et 43,9% de valencène non réagi (le restant étant de l'époxyde de valencène). Ceci correspond à un rendement en nootkatone d'environ 27,3 g par kg de mélange réactionnel.
Le produit de la réaction susmentionné a été pris dans 150 ml d'hexane et traité avec 75 g de Kieselgel 60 (origine : Merck). Après filtration et lavage à l'hexane (2 fois avec 75 ml), on a concentré les extraits pour obtenir 3,61 g de valencène pur à 86%. La désorption du silica gel a fourni 4,75 g de mélange contenant 43% de nootkatone, ce qui correspond à un rendement de 29,9% basé sur le valencène.
Un essai identique mais en utilisant une quantité plus élevée d'acide gras, à savoir 6 g, a fourni, après 24 h de réaction et le traitement usuel, 9,55 g d'un produit contenant 16,1% de nootkatone, 13,5% de nootkatol et 41,6% de valencène non réagi. Ceci correspond à un rendement de 21,5 g de nootkatone et 18,0 g de nootkatol, par kg de mélange réactionnel. Ces produits ont été obtenus à l'état pur par chromatographie sur SiO₂, en utilisant un mélange hexane/éther 9 : 1 en tant qu'éluant.

### Exemple 2

Un mélange de 46,4 g d'eau, 3,6 g de solution aqueuse de NaOH à 10%, 10,0 g de valencène et 4,0 g d'acide gras polyinsaturé a été doucement agité dans un ballon à 6 cols équipé d'un agitateur magnétique, d'un thermomètre, d'un appareil pour le contrôle du pH, d'une ampoule d'introduction et d'une entrée d'oxygène. Le mélange a été dégazé et purgé à vide (3 fois), et l'atmosphère remplacée par de l'oxygène pur. Une solution de 600 mg de lipoxygénase Type I-S dans 5 g de tampon de phosphate sodique à pH 8,5, contenue dans l'ampoule d'introduction, a été ajoutée en une seule fois et l'agitation augmentée à 750 rpm. Le pH a été automatiquement (PHstat) maintenu à 8,0 pendant toute la réaction par addition d'une solution aqueuse de NaOH à 5%. Une légère surpression de O₂ a été maintenue pendant toute la réaction pour permettre l'échantillonnage sans contamination d'air. La température a été maintenue à 60°.
Après 24 h, le mélange réactionnel a été refroidi à température ambiante, extrait à l'éther (3 x 200 ml), lavé avec une solution aqueuse de NaOH à 10% et ensuite à neutralité avec de la saumure. Les phases organiques combinées ont été séchées sur Na₂SO₄ et concentrées sous vide. Le produit ainsi obtenu (8,86 g) contenait 41,7% de nootkatone, 9,9% de nootkatol et 13,4% de valencène non réagi. Ceci correspond à la formation de 53,1 g de nootkatone par kg de mélange réactionnel.

### Exemple 3-29

Le valencène a été oxydé selon la méthode décrite à l'Exemple 1 ou 2, en variant les paramètres de la réaction comme il est indiqué dans le Tableau ci-après. Les concentrations ou rendements sont indiquées en g par kg de mélange réactionnel ou en % du produit obtenu. Toutes les réactions sont effectuées sous atmosphère d'oxygène pur, sauf indication contraire. L'acide gras employé était un hydrolysat d'huile de tournesol tel que cité à l'exemple 1, sauf indication contraire.
La valeur de pH indiquée correspond au pH initial du milieu, non-ajusté pendant la durée de la réaction, sauf indication contraire.

**TABLEAU I**

| Essai | Conc. de valencène g/kg | Lipoxygénase (source ou purifiée) | Acide gras g/kg | pH | Temp. °C | Temps de réaction h | Valencène non réagi | Rendement en nootkatone | | Rendement en nootkatol | |
|---|---|---|---|---|---|---|---|---|---|---|---|
| | | | | | | | % | g/kg | % | g/kg | % |
| 3 | 10,9 | farine de soja | - | 8,5 | ∼ 25 | 24 | 89,0 | 0,16 | 1,47 | 0,17 | 1,52 |
| 4 | 10,9 | farine de soja dégraissée | - | 8,5 | ∼ 25 | 24 | 98,2 | 0,02 | 0,20 | 0,02 | 0,22 |
| 5 | 10,9 | farine de soja | 4,3 | 8,5 | ∼ 25 | 24 | 81,5 | 0,33 | 3,03 | 0,12 | 1,09 |
| 6 | 46,6 | Type I-S ^{a)} | - | 8,5 | ∼ 25 | 24 | 100,0 | - | - | - | - |
| 7 | 46,6 | Type I-S ^{a)} | 18,6 | 8,5 | ∼ 25 | 24 | 77,0 | 1,12 | 2,40 | 0,67 | 1,43 |
| 8 | 46,6 | Type I-B ^{a)} | 18,6 | 8,5 | ∼ 25 | 24 | 82,0 | 1,17 | 2,50 | 0,61 | 1,30 |
| 9* | 46,6 | Type I-S ^{a)} | 18,6 | 8,5 | ∼ 25 | 24 | 92,9 | 0,15 | 0,32 | 0,09 | 0,19 |
| 10 | 46,6 | Type I-S ^{a)} | 18,6 | 6,5 | ∼ 25 | 48 | 81,3 | 1,11 | 2,37 | 0,68 | 1,45 |
| 11 | 46,6 | Type I-S ^{a)} | 18,6 | 7,0 ^{c)} | ∼ 25 | 48 | 79,6 | 1,10 | 2,35 | 0,76 | 1,64 |
| 12 | 46,6 | Type I-S ^{a)} | 18,6 | 7,5 ^{c)} | ∼ 25 | 48 | 78,3 | 1,34 | 2,86 | 0,79 | 1,70 |
| 13 | 46,6 | Type I-S ^{a)} | 18,6 | 8,0 ^{c)} | ∼ 25 | 48 | 82,8 | 1,67 | 3,58 | 0,87 | 1,86 |
| 14 | 46,6 | Type I-S ^{a)} | 18,6 | 8,5 ^{c)} | ∼ 25 | 48 | 79,6 | 1,62 | 3,47 | 0,94 | 2,01 |
| 15 | 46,6 | Type I-S ^{a)} | 18,6 | 9,0 ^{c)} | ∼ 25 | 48 | 77,9 | 1,62 | 3,47 | 0,85 | 1,83 |
| 16 | 143,7 | Type I-S ^{b)} | 57,5 | 8,5 | 15 | 24 | 89,5 | 1,08 | 0,75 | 0,93 | 0,65 |
| 17 | 143,7 | Type I-S ^{b)} | 57,5 | 8,5 | 25 | 24 | 85,7 | 2,26 | 1,57 | 1,52 | 1,06 |
| 18 | 143,7 | Type I-S ^{b)} | 57,5 | 8,5 | 35 | 24 | 82,0 | 3,68 | 2,56 | 3,12 | 2,17 |
| 19 | 143,7 | Type I-S ^{b)} | 57,5 | 8,5 | 40 | 24 | 73,5 | 4,53 | 3,15 | 3,97 | 2,76 |
| 20 | 143,7 | Type I-S ^{b)} | 57,5 | 8,5 | 45 | 24 | 75,0 | 6,51 | 4,53 | 5,43 | 3,78 |
| 21 | 143,7 | Type I-S ^{b)} | 57,5 | 8,5 | 50 | 24 | 58,5 | 9,47 | 6,59 | 7,16 | 4,98 |
| 22 | 143,7 | Type I-S ^{b)} | 57,5 | 8,5 | 60 | 24 | 46,3 | 26,94 | 18,75 | 14,63 | 10,18 |
| 23 | 143,7 | - | - | 8,5 | 60 | 24 | 89,9 | 0,12 | 0,08 | 0,08 | 0,05 |
| 24 | 143,7 | - | 57,9 | 8,5 | 60 | 24 | 47,0 | 19,25 | 13,28 | 11,30 | 7,80 |
| 25** | 143,7 | Type I-S ^{b)} | - | 8,5 | 60 | 24 | 53,0 | 18,76 | 13,41 | 11,44 | 8,18 |
| 26 | 143,7 | Type I-S ^{b)} | 57,5 | 8,5 | 70 | 24 | 30,0 | 30,53 | 21,25 | 14,22 | 9,90 |
| 27 | 143,7 | Type I-S ^{b)} | 57,5*** | 8,5 | 70 | 24 | 60,6 | 10,47 | 7,29 | 7,87 | 5,48 |
| 28 | 143,7 | Type I-S ^{b)} | 57,5 | 8,0 ^{c)} | 70 | 24 | 14,0 | 57,48 | 40,01 | 13,62 | 9,48 |
| 29 | 143,7 | Type I-S ^{b)} | 57,5 | 8,0 | 70 | 24 | 28,4 | 34,71 | 24,16 | 13,66 | 9,51 |

| | | | | | | | | | | | |
|---|---|---|---|---|---|---|---|---|---|---|---|
| * atmosphère d'air | | | | | | | | | | | |
| ** addition de 91,2 g d'acide 13-hydropéroxylinoléique par kg de mélange réactionnel | | | | | | | | | | | |
| *** acide linolénique | | | | | | | | | | | |
| a) 2,79 g/kg de mélange réactionnel | | | | | | | | | | | |
| b) 8,62 g/kg de mélange réactionnel | | | | | | | | | | | |
| c) pH maintenu pendant la réaction | | | | | | | | | | | |

### Exemple 30

A. On a préparé séparément l'hydropéroxyde d'acide gras comme suit: dans un ballon à 5 cols, équipé d'un agitateur mécanique, d'une entrée de O₂, d'une sonde pour contrôle du pH, une ampoule d'introduction et une introduction de poudre, on a chargé 262,0 g d'eau, 10,3 g de NaOH à 10% dans l'eau et 33,7 g de Nouracid HE-30, et on a mélangé à 20°. On a ajusté le pH à 9,5, valeur à laquelle il a été maintenu pendant toute la réaction, par addition d'une solution aqueuse de NaOH à 10%. On a purgé 3 fois à l'oxygène et ensuite ajouté 33,0 g de farine de soja fraîchement broyée. On a laissé réagir pendant 1 h, à la fin de laquelle l'analyse par titration iodométrique d'un aliquot de 500 mg (système titration : Na₂S₂O₃ 0,01 M, KI, CH₃COOH/CHCl₃ 2:3) du mélange réactionnel indiquait un rendement en hydropéroxyde, à savoir l'acide 13-hydropéroxylinoléique, de 88%, correspondant à un contenu de 63,0g d'hydropéroxyde par kg de mélange réactionnel.
   La suspension de l'hydropéroxyde ainsi obtenue a été décantée (alternativement, on peut séparer le solide par filtration et recueillir les filtrats) pendant 1 h et le liquide décanté séparé. On a lavé la farine restante à l'eau (306 g), agité pendant 1 min, et laissé décanter à nouveau pendant 1 h. Le liquide décanté a été à nouveau séparé et ajouté au premier liquide obtenu auparavant. La solution ainsi obtenue, contenant 32g d'hydropéroxyde par litre de solution (filtration) a été utilisée dans l'oxydation du valencène, comme décrit ci-après.
B. Dans un ballon à 5 cols, équipé d'une agitation mécanique, d'une entrée d'oxygène, d'un thermomètre et d'un dispositif de contrôle du pH (électrode pH et dispositif d'addition automatique de NaOH), chauffé à l'aide d'un bain d'huile, on a chargé 44 g de la solution d'hydropéroxyde préparée en A, 6,0 g de valencène et 1 goutte de produit antimousse (Dow Coming® FG-10). On a purgé 4 fois à l'oxygène, ajusté le pH à 8 et la température à 70°. On a laissé réagir en maintenant le pH à 8 comme indiqué à l'Exemple 1. L'évolution de la réaction a été suivie par chromatographie en phase gazeuse d'aliquots pris et analysés comme indiqué à l'Exemple 1.
   Ces analyses (colonne de type Spb-1, 15 m, 100° 1 min, puis 100-160° à 5°/min, temps rétention de la nootkatone 12,63 min) indiquaient un contenu de 34,56% en poids de nootkatone et de 9,47% de nootkatol dans le produit de la réaction, après 24 h, alors que ces valeurs étaient respectivement de 44,43% et 7,94% après 48 h de réaction.
   Après 48 h, on a stoppé la réaction, ajusté le pH à 12, extrait avec 125 ml de cyclohexane, ensuite avec 125 ml de cyclohexane plus 10% de NaCl. Les phases organiques ont été lavées à neutralité à l'aide de 2 fois 50 ml de H₂O + 1% NaCI. La solution (environ 300 ml) a été passée sur 22 g de SiO₂ (silice Chromagel® 60, 70-200 µm) et le produit distillé au four à boules (150°/10 Pa) pour fournir environ 3,2 g de produit concentré ( [α]²⁰_{D} = +129,4° (2% dans le CHCl₃)).
   Le rendement en nootkatone après 48 h de réaction et séparation du milieu de réaction était de 53,3 g par litre de mélange réactionnel.

### Exemples 31 - 44

On a procédé comme il est décrit à l'Exemple 2 ou 30, c'est-à-dire en préparant l'hydropéroxyde in-situ, respectivement séparément, mais en variant soit l'acide gras de départ, soit les conditions de réaction, comme indiqué dans le Tableau ci-après. Toutes les réactions ont été effectuées sous atmosphère d'oxygène pur, à un pH constant égal à 8 et une température de 70°, pendant 48 h. Les résultats sont indiqués dans les Tableaux ci-après. Les concentrations indiquées s'entendent par rapport au mélange réactionnel et avant séparation de ce dernier.
Lorsque les hydropéroxydes ont été préparés par photooxydation des acides gras correspondants, les conditions de réaction étaient similaires à celles décrites précédemment, avec la seule différence que le mélange d'acide gras et de sensibilisateur (Rose bengale) a été irradié sous atmosphère d'oxygène, à l'aide d'une lampe Philips HPK 125 W BA 15 D Type 57203 B9.

**TABLEAU II**

| Essai | Mode d'exécution | Conc. de valencène g/kg | Catalyseur mg/kg | Acide gras ou hydropéroxyde g/kg | Rendement en nootkatone g/kg |
|---|---|---|---|---|---|
| 31 | Ex. 2 | 156,1 | Cu stéarate (781) | Acide linoléique (40,3) | 37,8 |
| 32 | Ex. 2 | 156,1 | Co naphténate (781) | Acide linoléique (40,3) | 55,2 |
| 33 | Ex. 2 | 156,1 | Cu poudre (781) | Acide linoléique (40,3) | 28,8 |
| 34 | Ex. 2 | 154,9 | Cu stéarate (775) | Acide oléique (10,1) | 56,2 |
| 35 | Ex. 2 | 154,9 | Cu naphténate (775) | Acide oléique (10,1) | 57,8 |
| 36 | Ex. 2 | 155,8 | Cu poudre (3115) | Acide oléique (40,5) | 41,0 |
| 37* | Ex. 30 | 161,3 | Rose bengale | Acide linoléique (40,1) | 39,5 |
| 38* | Ex. 30 | 161,3 | Rose bengale | Acide oléique (40,4) | 47,1 |
| 39 | Ex. 30 | 153,8 | - | 13-HPOD (16,2) | 56,6 |
| 40 | Ex. 2 | 172,2 | Farine de soja (86'580) | Acide linoléique (11,2) | 38,0 |
| 41 | Ex. 30 | 153,8 | - | 13-HPOD** ¹⁾ (27,7) | 41,2 |
| 42 | Ex. 30 | 120,0 | - | 13-HPOD** ²⁾ (27,3) | 55,0 |
| 43 | Ex. 30 | 120,0 | - | 13-HPOT** (27,0) | 47,6 |
| 44 | Ex. 30 | 120,0 | - | 13-HPOD** ³⁾ (20,2) | 14,5 |

| | | | | | |
|---|---|---|---|---|---|
| * essai utilisant des hydropéroxydes préparés par photooxydation | | | | | |
| ** 13-HPOD = acide 13-hydropéroxylinoléique 13-HPOT = acide 13-hydropéroxylinolénique | | | | | |
| 1) sol. 13-HPOD décantée | | | | | |
| 2) sol. 13-HPOD filtrée | | | | | |
| 3) sol. 13-HPOD centrifugée | | | | | |

## Revendications

1. Procédé pour la préparation de la nootkatone ou du nootkatol, ou des mélanges de ces composés, par oxydation du valencène, caractérisé en ce qu'on expose ce dernier à une atmosphère d'oxygène dans un milieu de réaction approprié et en présence d'un hydropéroxyde d'un acide gras insaturé et en ce que, le cas échéant, on sépare la nootkatone et/ou le nootkatol du mélange réactionnel.

2. Procédé selon la revendication 1, caractérisé en ce que l'acide gras est polyinsaturé.

3. Procédé selon la revendication 1 ou 2, caractérisé en ce que l'oxydation du valencène a lieu sous atmosphère d'oxygène pur.

4. Procédé selon la revendication 2, caractérisé en ce que l'oxydation du valencène a lieu en présence d'un hydropéroxyde de l'acide linoléique ou de l'acide linolénique.

5. Procédé selon la revendication 1, caractérisé en ce que l'oxydation du valencène a lieu en présence d'un hydropéroxyde de l'acide oléique.

6. Procédé selon l'une des revendications 1 à 5, caractérisé en ce que ledit hydropéroxyde est engendré in situ dans le milieu de réaction à partir de l'acide gras insaturé approprié.

7. Procédé selon la revendication 6, caractérisé en ce que l'acide gras insaturé est l'acide oléique, l'acide linoléique, l'acide linolénique ou l'un de leurs précurseurs d'origine naturelle.

8. Procédé selon la revendication 6 ou 7, caractérisé en ce qu'on ajoute au milieu de réaction un catalyseur susceptible de promouvoir la formation dudit hydropéroxyde.

9. Procédé selon la revendication 8, caractérisé en ce qu'on utilise un acide gras polyinsaturé et en ce qu'on ajoute au milieu de réaction une lipoxygénase.

10. Procédé selon la revendication 8, caractérisé en ce que le catalyseur est le cuivre en poudre, le naphténate de cuivre ou le stéarate de cuivre.

11. Procédé selon la revendication 8, caractérisé en ce que l'hydropéroxyde est engendré par photooxydation de l'acide gras insaturé en présence de Rose bengale.

12. Procédé selon l'une des revendications précédentes, caractérisé en ce que la température du milieu de réaction est maintenue à une valeur comprise entre 20 et 80°C.

13. Procédé selon la revendication 12, caractérisé en ce que la température est de l'ordre de 50°C ou plus.

14. Procédé selon l'une des revendications précédentes, caractérisé en ce que le pH du milieu de réaction a une valeur comprise entre 5 et 10.

15. Procédé selon la revendication 14, caractérisé en ce que le pH a une valeur d'environ 8 et ce qu'on obtient essentiellement la nootkatone.

16. Procédé selon la revendication 1, caractérisé en ce que le valencène est utilisé sous forme de l'un de ses isomères optiquement actifs et en ce que l'on obtient l'isomère optiquement actif correspondant de la nootkatone et/ou du nootkatol.

17. Procédé selon la revendication 1, caractérisé en ce que l'oxydation du valencène est effectuée sous atmosphère d'oxygène pur et à une température comprise entre 50 et 80°C, le pH du milieu de réaction étant maintenu à une valeur comprise entre 8 et 9,5.

## Patentansprüche

1. Verfahren zur Herstellung von Nootkaton oder Nootkatol bzw. von Mischungen dieser Verbindungen mittels Oxidation von Valencen, dadurch gekennzeichnet, daß das Valencen in einem geeigneten Reaktionsmedium und in Gegenwart eines Hydroperoxids einer ungesättigten Fettsäure einer Sauerstoffatmosphäre ausgesetzt wird, sowie dadurch, daß das Nootkaton und/oder Nootkatol gegebenenfalls vom Reaktionsmedium getrennt wird.

2. Verfahren nach Anspruch 1, dadurch gekennzeichnet, daß die Fettsäure mehrfach ungesättigt ist.

3. Verfahren nach Anspruch 1 oder 2, dadurch gekennzeichnet, daß die Oxidation von Valencen unter reiner Sauerstoffatmosphäre stattfindet.

4. Verfahren nach Anspruch 2, dadurch gekennzeichnet, daß die Oxidation von Valencen in Gegenwart eines Hydroperoxids von Linolsäure oder Linolensäure stattfindet.

5. Verfahren nach Anspruch 1, dadurch gekennzeichnet, daß die Oxidation von Valencen in Gegenwart eines Hydroperoxids von Ölsäure stattfindet.

6. Verfahren nach einem der Ansprüche 1 bis 5, dadurch gekennzeichnet, daß das Hydroperoxid ausgehend von der geeigneten ungesättigten Fettsäure *in situ* in dem Reaktionsmedium erzeugt wird.

7. Verfahren nach Anspruch 6, dadurch gekennzeichnet, daß die ungesättigte Fettsäure Ölsäure, Linolsäure, Linolensäure oder einer deren Vorläufer natürlicher Herkunft ist.

8. Verfahren nach Anspruch 6 oder 7, dadurch gekennzeichnet, daß dem Reaktionsmedium ein Katalysator zugegeben wird, der in der Lage ist, die Bildung des Hydroperoxids zu fördern.

9. Verfahren nach Anspruch 8, dadurch gekennzeichnet, daß eine mehrfach ungesättigte Fettsäure verwendet wird, und daß dem Reaktionsmedium eine Lipoxygenase zugesetzt wird.

10. Verfahren nach Anspruch 8, dadurch gekennzeichnet, daß der Katalysator pulverförmiges Kupfer, Kupfernaphthenat oder Kupferstearat ist.

11. Verfahren nach Anspruch 8, dadurch gekennzeichnet, daß das Hydroperoxid mittels Photooxidation der ungesättigten Fettsäure in Gegenwart von Bengalrosa erzeugt wird.

12. Verfahren nach einem der vorhergehenden Ansprüche, dadurch gekennzeichnet, daß die Temperatur des Reaktionsmediums bei einem zwischen einschließlich 20 und 80°C liegenden Wert gehalten wird.

13. Verfahren nach Anspruch 12, dadurch gekennzeichnet, daß die Temperatur in der Größenordnung von 50°C oder mehr ist.

14. Verfahren nach einem der vorhergehenden Ansprüche, dadurch gekennzeichnet, daß der pH des Reaktionsmediums einen zwischen einschließlich 5 und 10 liegenden Wert aufweist.

15. Verfahren nach Anspruch 14, dadurch gekennzeichnet, daß der pH einen Wert von etwa 8 aufweist, und daß im wesentlichen Nootkaton hergestellt wird.

16. Verfahren nach Anspruch 1, dadurch gekennzeichnet, daß das Valencen in Form eines seiner optisch aktiven Isomere verwendet wird, und daß das entsprechende optisch aktive Isomer von Nootkaton und/oder Nootkatol hergestellt wird.

17. Verfahren nach Anspruch 1, dadurch gekennzeichnet, daß die Oxidation von Valencen unter reiner Sauerstoffatmosphäre und bei einer zwischen einschließlich 50 und 80°C liegenden Temperatur durchgeführt wird, wobei der pH des Reaktionsmediums auf einem zwischen einschließlich 8 und 9,5 liegenden Wert gehalten wird.

## Claims

1. Process for the preparation of nootkatone or nootkatol, or of mixtures of these compounds, by oxidation of valencene, characterized in that the latter is exposed to an oxygen-containing atmosphere in an appropriate reaction medium and in the presence of a hydroperoxyde of an unsaturated fatty acid and in that, optionally, the nootkatone and/or the nootkatol is separated from the reaction mixture.

2. Process according to claim 1, characterized in that the fatty acid is a polyunsaturated fatty acid.

3. Process according to claim 1 or 2, characterized in that the oxidation of valencene is carried out under a pure oxygen atmosphere.

4. Process according to claim 2, characterized in that the oxidation of valencene is carried out in the presence of a hydroperoxyde of linoleic or of linolenic acid.

5. Process according to claim 1, characterized in that the oxidation of valencene is carried out in the presence of a hydroperoxyde of oleic acid.

6. Process according to any one of claims 1 to 5, characterized in that said hydroperoxyde is formed in situ in the reaction medium from the appropriate unsaturated fatty acid.

7. Process according to claim 6, characterized in that the unsaturated fatty acid is oleic acid, linoleic acid, linolenic acid or one of their natural origin precursors.

8. Process according to claim 6 or 7, characterized in that there is added to the reaction medium a catalyst susceptible of promoting the formation of said hydroperoxyde.

9. Process according to claim 8, characterized in that a polyunsaturated fatty acid is used and in that a lipoxygenase is added to the reaction medium.

10. Process according to claim 8, characterized in that the catalyst is copper powder, copper naphthenate or copper stearate.

11. Process according to claim 8, characterized in that the hydroperoxyde is formed by photooxidation of the unsaturated fatty acid in the presence of Rose bengale.

12. Process according to any one of the preceding claims. characterized in that the temperature of the reaction medium is maintained at a value comprised between 20 and 80°C.

13. Process according to claim 12, characterized in that the temperature is of the order of 50°C or higher.

14. Process according to any one of the preceding claims, characterized in that the pH of the reaction medium has a value comprised between 5 and 10.

15. Process according to claim 14, characterized in that the pH has a value of about 8 and in that essentially nootkatone is obtained.

16. Process according to claim 1, characterized in that valencene is used in the form of one of its optically active isomers and in that the corresponding optically active isomer of nootkatone and/or nootkatol is obtained.

17. Process according to claim 1, characterized in that the oxidation of valencene is carried out under a pure oxygen atmosphere and at a temperature comprised between 50 and 80°C, the pH of the reaction medium being maintained at a value comprised between 8 and 9.5.
